# EUROPÄISCHE PATENTSCHRIFT

(11) **EP 3 701 890 B1**
(45) Veröffentlichungstag und Bekanntmachung des Hinweises auf die Patenterteilung: **10.09.2025**
(21) Anmeldenummer: 20150833.0
(22) Anmeldetag: 09.01.2020
(51) Int. Cl.: A61B 17/34, A61F 9/007

(54) **EIN OPHTHALMOLOGISCHES HANDGERÄT SOWIE EIN SET UMFASSEND EIN OPHTHALMOLOGISCHES HANDGERÄT**
AN OPHTHALMOLOGICAL HAND-HELD DEVICE AND A SET COMPRISING AN OPHTHALMOLOGICAL HAND-HELD DEVICE
APPAREIL OPHTALMOLOGIQUE PORTATIF AINSI QU'ENSEMBLE COMPRENANT UN APPAREIL OPHTALMOLOGIQUE PORTATIF

(30) Priorität: 18.02.2019 DE 102019202158
(43) Veröffentlichungstag der Anmeldung: 02.09.2020
(73) Patentinhaber: Geuder AG, 69126 Heidelberg (DE)
(72) Erfinder: El-Ayari, Hamadi, 60323 Frankfurt (DE); Draheim, René, 69207 Sandhausen (DE); Pelka, Falko, 75050 Gemmingen (DE)
(74) Vertreter: Ullrich & Naumann PartG mbB

(56) Entgegenhaltungen:
- DE-U1- 202012 013 057
- DE-U1- 29 912 897
- US-A1- 2006 276 738
- US-A1- 2010 100 058
- US-A1- 2013 102 966
- US-A1- 2016 015 563

## Beschreibung

Die vorliegende Erfindung betrifft ein ophthalmologisches Handgerät zum Einbringen einer Inzision in das menschliche oder tierische Auge, insbesondere Trokarlanze, mit einem Griffstück und einem Schaft, wobei an einem distalen Ende des Schafts eine Spitze ausgebildet ist. Des Weiteren betrifft die Erfindung ein Set bestehend aus einem solchen ophthalmologischen Handgerät und mindestens zwei Trokaren.

Bei ophthalmologischen Eingriffen ist es üblich, eine Inzision in Form eines Einstichkanals in dem Auge anzubringen, um gleichzeitig oder darauf folgend einen Trokar in dem Einstichkanal zu platzieren. Der Trokar dient dabei als Zugang für chirurgische Instrumente oder zum Einführen von Medien (beispielsweise Infusionen) oder Licht in das Auge des Patienten. Durch den Trokar werden die Wundränder geschont und es kann eine gewisse Abdichtung des Augeninneren gegen die Außenumgebung erreicht werden.

Mit anderen Worten sind Trokare Vorrichtungen zum Einführen eines Mediums oder eines Instruments in das Auge eines Patienten. Solche Vorrichtungen sind beispielsweise in dem Dokument EP 1 886 653 A1 beschrieben.

Im Rahmen entsprechender Eingriffe werden regelmäßig mehrere Trokare durch die Sklera hindurch in das Auge eingeführt. Üblicherweise wird ein als Führungsrohr dienender ersten Trokar derart in das Auge eingesetzt, dass die Spitze der Sonde des Trokars bis in den Glaskörperraum des Auges hineinreicht. Das von der Sonde abgewandte Ende des Trokars, d. h. der Anschlusskörper, dient auf der der Sonde abgewandten Seite zum Anschließen eines Schlauchs, über den das Innere des Auges mit einer Infusionsflüssigkeit versorgbar ist. Dieser Trokar wird üblicherweise auch als Infusionstrokar bezeichnet.

Des Weiteren ist es üblich, ein oder zwei weitere Trokare in das Auge einzusetzen, über die sich die benötigten Instrumente, beispielsweise ein Vitrektor, ein Zängchen oder eine Beleuchtungseinrichtung, in das Auge einführen lassen. Diese Trokare werden üblicherweise als Instrumententrokare bzw. als Beleuchtungstrokare bezeichnet.

Die hier in Rede stehenden Trokare haben den Vorteil, dass diese in eine kleine Öffnung der Sklera eingesetzt werden und dass die Instrumente durch den jeweiligen Trokar hindurch austauschbar sind. Eine Reizung oder Beschädigung des Auges wird durch die Verwendung des Trokars vermieden.

Zum Setzen des Trokars kann beispielsweise mit einem Skalpell oder mit einer Kanüle einer Inzision eingebracht werden, durch welche darauffolgend der Trokar gesteckt werden kann. Des Weiteren sind Handgeräte zum Setzen eines Trokars vorbekannt, die eine dornartige Spitze aufweisen und als Trokarlanze bezeichnet werden. Mit seiner solchen Trokarlanze kann der Trokar in der Inzision verankert werden. Hierzu wird üblicherweise der Trokar zunächst auf einen schafkantigen Abschnitt des Handinstruments aufgeschoben und nach dem Einbringen der Lanzenspitze von dieser in die Öffnung des Auges geschoben. Typischerweise werden drei Trokarlanzen mit jeweils einem darauf aufgefädelten Trokar steril verpackt ausgeliefert, sodass dem Operateur von einer Hilfsperson dreimal eine Trokarlanze mit darauf aufgeschobenem Trokar angereicht wird, um die beispielsweise bei einer Pars-Plana-Vitrektomie benötigten drei Trokare zu setzen. Folglich ist der anfallende Materialaufwand hoch, zumal es sich im Regelfall um Einwegprodukte handelt und zumal die Sterilverpackung aufwändig im Aufbau und der Herstellung ist. Daher sind die aus dem Stand der Technik bekannten Vorrichtungen äußerst unwirtschaftlich. Des Weiteren ist die Handhabung für den Operateur kompliziert, da er mehrmals ein Instrument angereicht bekommt und somit auch ein mehrfaches Umgreifen an dem Handgerät notwendig ist.

Dokumente DE 20 2012 013057 U1, US 2013/102966 A1 und US 2016/015563 A1 offenbaren ophthalmologische Handgeräte zum Einbringen von Inzisionen und mehreren Trokaren in das menschliche oder tierische Auge, mit unterschiedlichen Magazinvorrichtungen zum Halten mehrerer Trokare.

Die vorliegenden Erfindungen liegt die Aufgabe zugrunde, ein ophthalmologisches Handgerät zum Einbringen einer Inzision in das menschliche oder tierische Auge der eingangs genannten Art derart auszugestalten weiterzubilden, dass mit konstruktiv einfachen Mitteln die Vorrichtung vereinfacht und deren Handhabung verbessert wird. Des Weiteren soll ein Set umfassend ein verbessertes ophthalmologisches Handgerät angegeben werden.

Erfindungsgemäß wird die zugrundeliegende Aufgabe durch ein ophthalmologisches Handgerät mit den Merkmalen des Anspruchs 1 gelöst, weitere Ausführungsformen werden in den abhängigen Ansprüchen offenbart. Danach ist das erfindungsgemäße ophthalmologische Handgerät dazu vorgesehen, dass eine Magazineinrichtung zum Vorhalten von mindestens zwei Trokaren darin ist.

In erfindungsgemäßer Weise ist zunächst erkannt worden, dass die zugrundeliegende Aufgabe in verblüffend einfacher Weise gelöst wird, wenn mehrere Trokare an dem ophthalmologischen Handgerät vorgehalten werden können. In weiter erfindungsgemäßer Weise ist hierzu eine Magazineinrichtung vorgesehen, welche zum Vorhalten der Trokare dient. Die Magazinvorrichtung könnte beispielsweise an und/oder in dem Griffstück vorgesehen sein. Ein mehrfaches Anreichen des Handgeräts an den Operateur sowie ein Umgreifen an dem Handgerät sind nicht mehr notwendig. Durch die erfindungsgemäßen konstruktiven Merkmale wird die Handhabung für das Setzen von mehreren Trokaren erheblich vereinfacht. Des Weiteren wird der Materialeinsatz und somit die Herstellungskosten erheblich minimiert, da lediglich ein einziges Handgerät benötigt wird und dessen Verpackung kleiner ausgebildet sein kann.

In vorteilhafter Weise kann die Magazineinrichtung derart ausgebildet sein, dass drei Trokare aufnehmbar sind. Durch eine solche Konstruktion ist das ophthalmologische Handgerät beispielsweise zur Durchführung einer Pars-Plana-Vitrektomie besonders geeignet, bei welcher drei verschiedene Trokare für die Beleuchtung, die Infusion und den Vitrektor gesetzt werden müssen.

Gemäß einer alternativen Ausgestaltung, die nicht unter den Gegenstand der beanspruchten Erfindung fällt, könnte die Magazineinrichtung als Revolvermagazin ausgebildet sein. Dabei ist es denkbar, dass das Revolvermagazin mindestens zwei, vorzugsweise drei Aufnahmen für einen Trokar aufweist. Bei einer solchen Konstruktion werden die Trokare besonders sicher gehalten, auch wenn diese beispielsweise eine seitliche Ausnehmung von größer als 180° aufweisen und somit nicht ausreichend auf einer üblichen Trokarlanze gehalten werden können. Des Weiteren könnte durch Drehen des Revolvermagazins jeweils eine der Aufnahmen fluchtend mit der Mittelachse des Schafts ausgerichtet werden. Somit kann durch ein Weiterdrehen des Revolvermagazins jeweils eine mit einem Trokar bestückte Aufnahme derart ausgerichtet werden, dass der Handgriff mit dem Trokar "geladen" wird und in die Inzision des Auges eingeschoben werden kann.

Erfindungsgemäß sind die Trokare hintereinander auf dem Schaft aufgeschoben, sodass der Schaft die Trokare vorhält und dadurch die Magazineinrichtung bildet. Eine solche Konstruktion ist besonders einfach und somit kostengünstig herstellbar. Wesentlich ist dabei lediglich, dass der Schaft eine ausreichende Länge aufweist, um die gewünschte Anzahl von Trokaren aufzunehmen.

Um die Trokare in Richtung des dem Auge zugewandten Endes des Griffstücks zu verbringen, ist eine Verbringeinrichtung vorgesehen. Die Verbringeinrichtung weist ein Schiebeelement mit einem Haltemittel für einen Trokar auf.

Das Haltemittel kann insbesondere als Anschlag ausgebildet sein, der an einer dem Auge abgewandten Seite des Grundkörpers des Trokars angreift bzw. an diesem hintergreift. Dadurch ist es auf einfache Weise möglich, den Trokar entlang der Längsrichtung des Handgeräts zu verschieben.

Gemäß einer Ausgestaltung, die nicht unter den Gegenstand der beanspruchten Erfindung fällt, könnte der Schaft, insbesondere in axialer Richtung, bewegbar ausgebildet sein und ein Haltemittel für einen Trokar aufweisen, sodass der Schaft als Schiebeelement dient. Eine solche Konstruktion eignet sich insbesondere in Kombination mit einem Revolvermagazin. Dabei kann eine mit einem Trokar versehene Aufnahme des Revolvermagazins fluchtend mit der Mittelachse des Schafts ausgerichtet werden. Der Schaft kann sodann in Richtung des dem Auge zugewandten Ende des Griffstücks geschoben werden, sodass der Trokar auf den Schaft aufgefädelt wird und mit dem Schaft in das Auge einbringbar ist.

Erfingungsgemäß erstreckt sich das Schiebeelement seitlich versetzt von der Mittelachse des Schafts. Eine solche Konstruktion eignet sich in Kombination mit dem als Magazineinrichtung dienenden Schaft, auf welchen Trokare bereits aufgesteckt sind. Wesentlich ist dabei, dass die Haltemittel des Schiebeelements an dem Trokar zumindest hintergreifen, sodass der Trokar bzw. die Trokare über die Verbringeinrichtung in Richtung des dem Auge zugewandten Ende des Griffstücks verschiebbar sind. Nachdem ein Trokar in eine Inzision des Auges eingeschoben ist, kann das Schiebeelement in vorteilhafter Weise im Wesentlichen in Radialrichtung von den Schaft weg bewegbar sein, sodass durch ein Wegdrücken und Zurückziehen des Schiebeelements der nächste Trokar "geladen" wird, d.h. vor dem Schiebelement angeordnet ist. In vorteilhafter Weise könnte das Schiebeelement in Richtung der Mittelachse des Schafts vorgespannt sein. Somit kann gewährleistet werden, dass die Haltemittel jeweils an den Trokaren angreifen können.

In besonders vorteilhafter Weise kann die Spitze des Schafts eine seitliche Ausnehmung aufweisen, sodass die Spitze einen von einem runden Querschnitt abweichenden Querschnitt aufweist. Insbesondere könnte die Ausnehmung derart ausgebildet sein, dass die Spitze einen mondförmigen bzw. kreisbogenförmigen Querschnitt aufweist. Hierzu könnte der Schaft entsprechend ausgefräst bzw. ausgeklinkt sein. Eine solche Konstruktion hat den Vorteil, dass sich die Vorrichtung insbesondere zum Setzen von Trokaren mit halbmondförmigen bzw. halbrohrförmigen Sonden eignet. Entsprechende Trokare haben den Vorteil, dass sie eine ideale Abdichtung des Augeninneren gegenüber der Umgebung ermöglichen. Der Begriff "Querschnitt" bezeichnet dabei einen Schnitt orthogonal zur Längsachse des Schafts.

Um auf dem runden Augapfel eine flache, selbstdichtende Wundarchitektur zu erzielen, kann die Spitze des Schafts in vorteilhafter Weise einen MVR-Schliff aufweisen.

Die zugrunde liegende Aufgabe wird des Weiteren auch durch ein Set gemäß Anspruch 7 gelöst. Danach besteht ein erfindungsgemäßes Set aus einem Handgerät nach einem der Ansprüche 1 bis 6 und mindestens zwei Trokaren, wobei die Trokare in und/oder an der Magazineinrichtung anordenbar sind.

In vorteilhafter Weise können die Trokare einen Anschlusskörper, einen im Anschlusskörper ausgebildeten Durchgang und eine sich an den Durchgang anschließende Sonde aufweisen. Die Sonde kann zum Einführen in das Augeninnere dienen, wobei der Anschlusskörper auf der der Sonde zugewandten Seite zur Anlage außerhalb des Auges und auf der der Sonde abgewandten Seite zum Einführen bzw. Ankoppeln eines Schlauchs, Instruments oder dergleichen dient. Um eine Abdichtung des Augeninneren zu ermöglichen, kann der Anschlusskörper eine sich vom Randbereich bis zum Durchgang bzw. zur Sonde erstreckende Ausnehmung oder Aussparung aufweisen, die sich zum Durchgang hin verjüngen kann. Die Ausnehmung kann sich über einen Winkel im Bereich von 45° bis 270°, vorzugsweise in der in einem Bereich von etwa 60° erstrecken. Insbesondere ist es denkbar, dass sich die Ausnehmung in die Sonde hinein und vorzugsweise durch die Sonde hindurch bzw. über deren gesamte Länge hinweg erstreckt.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Offenbarung anhand der Zeichnung zu verweisen. In Verbindung mit der Erläuterung der bevorzugten Ausführungsbeispiele der Offenbarung anhand der Zeichnung werden auch im Allgemeinen bevorzugte Ausgestaltungen und Weiterbildungen der Lehre erläutert.

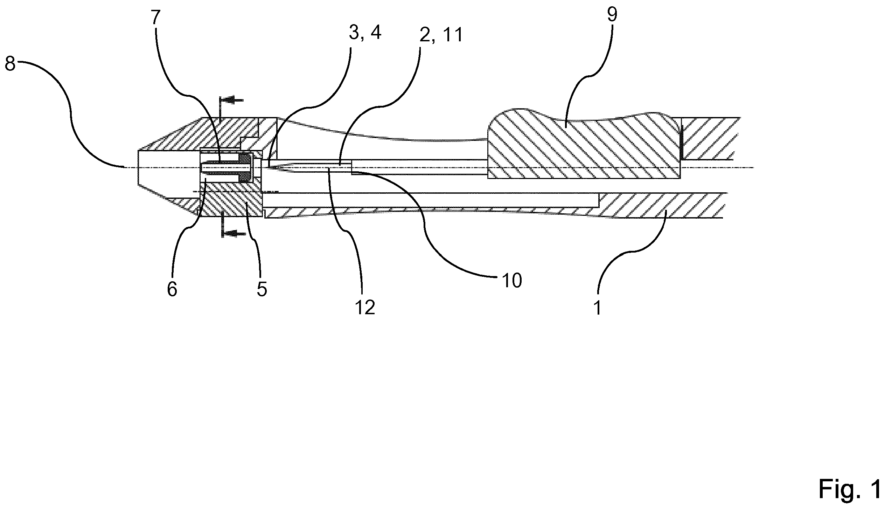

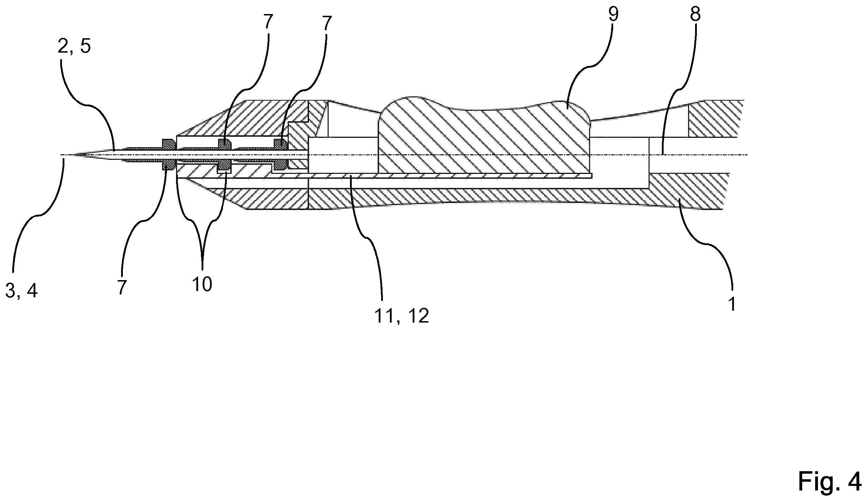

Die Erfindung ist im beigefügten Anspruchssatz beschrieben. Die Figuren 4- 7 zeigen ein Ausführungsbeispiel entsprechend der beanspruchten Erfindung. Das in Figuren 1- 3 gezeigte alternative Ausführungsbeispiel der Offenbarung, fällt nicht unter den Schutzumfang der beanspruchten Erfindung. In der Zeichnung zeigen
- Fig. 1: in einer schematischen, geschnittenen Darstellung ein Ausführungsbeispiel eines Handgeräts,
- Fig. 2: in einer weiteren schematischen, geschnittenen Darstellung das Ausführungsbeispiel gemäß Fig. 1,
- Fig. 3: in einer schematischen Darstellung einen Schnitt durch das Handgerät gemäß Fig. 1
- Fig. 4: in einer schematischen, geschnittenen Darstellung ein Ausführungsbeispiel eines erfindungsgemäßen Handgeräts,
- Fig. 5: in einer weiteren schematischen, geschnittenen Darstellung das Ausführungsbeispiel gemäß Fig. 4,
- Fig. 6: in einer weiteren schematischen, geschnittenen Darstellung das Ausführungsbeispiel gemäß Fig. 4,
- Fig. 7: in einer weiteren schematischen, geschnittenen Darstellung das Ausführungsbeispiel gemäß Fig. 4,
- Fig. 8: in einer weiteren schematischen, geschnittenen Darstellung das Ausführungsbeispiel gemäß Fig. 4,
- Fig. 9: in einer schematischen, perspektivischen Ansicht ein Ausführungsbeispiel eines Trokars zur Verwendung mit einem erfindungsgemäßen Handgerät, und
- Fig. 10: in einer weiteren schematischen, perspektivischen Ansicht den Trokar gemäß Fig. 9.

Die Fig. 1 bis 3 zeigen ein erstes Ausführungsbeispiel eines ophthalmologischen Handgeräts der Offenbarung, welches nicht Teil der beanspruchten Erfindung ist. Das Handgerät dient zum Einbringen einer Inzision in das menschliche oder tierische Auge und umfasst ein Griffstück 1 und einen Schaft 2. An dem distalen Ende 3 des Schafts 2 ist eine Spitze 4 ausgebildet, die zum Einbringen der Inzision an dem Auge dient. Des Weiteren ist in den Griffstück eine Magazineinrichtung 5 vorgesehen, die in diesem Ausführungsbeispiel als Revolvermagazin realisiert ist.

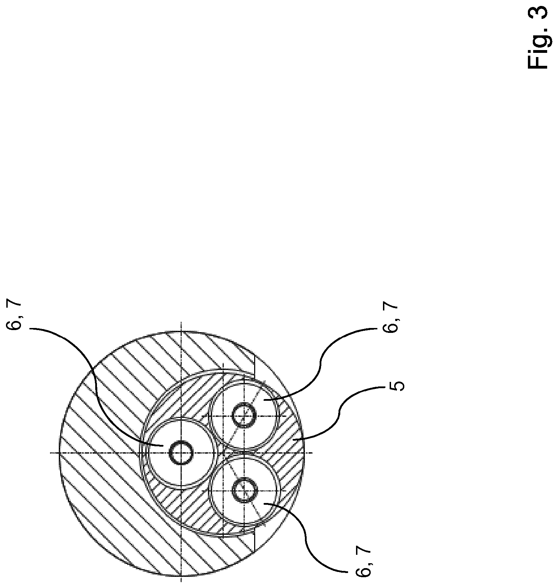

Insbesondere in Fig. 3 ist deutlich zu erkennen, dass die Magazineinrichtung 5 drei Aufnahmen 6 aufweist. In jeder der Aufnahmen 6 kann ein Trokar 7 eingebracht werden. Durch Drehen des Revolvermagazins 5 kann jeweils eine Aufnahme 6 mit dem darin angeordneten Trokar 7 fluchtend mit der Mittelachse 8 des Schafts 2 ausgerichtet werden. Der Schaft 2 ist in Längsrichtung des Griffstücks 2 verschiebbar, nämlich über ein mit dem Schaft 2 verbundenes Betätigungselement 9. In dem hier gezeigten Ausführungsbeispiel ist das Betätigungselement 9 als Knopf ausgebildet.

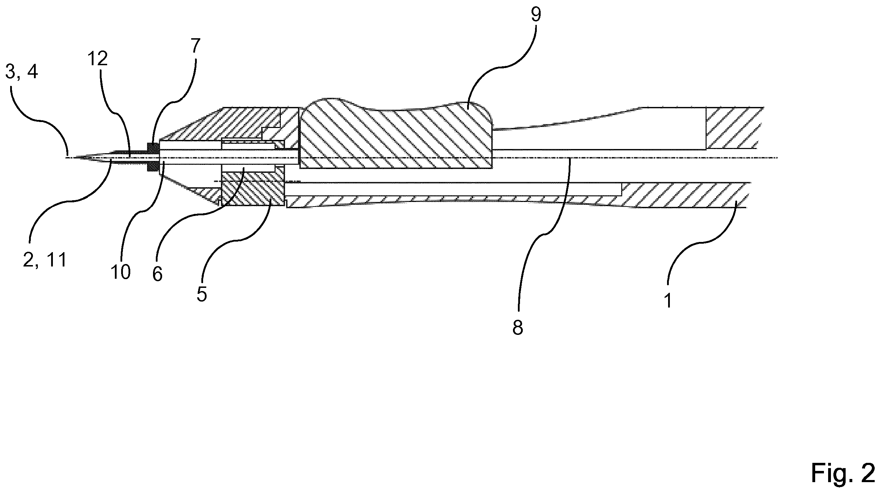

Wenn der Schaft 2 von der in Fig. 1 dargestellten Position in die in Fig. 2 dargestellte Position verbracht wird, wird der Trokar 7 auf den Schaft aufgeschoben bzw. aufgefädelt, wobei an dem Schaft 2 ein Haltemittel 10 in Form eines Anschlags ausgebildet ist, sodass der Trokar 7 nicht zu weit über den Schaft 2 rutschen kann und von diesem mitgenommen wird. Somit dient der Schaft 2 als Schiebeelement 11 einer Verbringeinrichtung 12. Fig. 2 zeigt den Zustand des Handgeräts mit auf dem Schaft 2 aufgeschobenem Trokar 7. Wenn sich das Handgerät in diesem Zustand befindet, kann der Chirurg die Spitze 4 in das Auge einstechen und sodann den Trokar 7 in die entstandene Inzision einführen.

Nachdem der erste Trokar 7 in das Auge gesetzt ist, kann der Schaft 2 in das Griffstück 1 zurückgezogen werden und das Revolvermagazin 5 weitergedreht werden, um den nächsten Trokar 7 vor den Schaft 2 zu "laden". Somit kann der Operateur nach und nach die Trokare 7 über den als Verschiebeelement 11 dienenden Schaft 2 in das Auge einbringen, ohne das Handgerät aus der Hand legen zu müssen. Zur Vorbereitung des Eingriffs ist lediglich das Revolvermagazin 5 mit den Trokaren 7 zu bestücken.

Die Fig. 4 bis 8 zeigen ein Ausführungsbeispiel eines erfindungsgemäßen ophthalmologischen Handgeräts. Dieses weist ein Griffstück 1 und einen Schaft 2 auf, an dessen distalem Ende 3 eine Spitze 4 ausgebildet ist. Fig. 1 ist zu entnehmen, dass drei Trokare 7 auf dem Schaft 2 aufgeschoben sind. Bei diesem Ausführungsbeispiel weist die Verbringeinrichtung 12 ein separates Schiebeelement 11 auf. Das Schiebeelement 11 erstreckt sich zeitlich versetzt von der Mittelachse 8 entlang des Schafts 2. Das Schiebeelement 11 ist über ein Betätigungselement 9 in Längsrichtung des Handgeräts hin und her bewegbar. An dem Schiebeelement 11 sind Haltemittel 10 für drei Trokare 7 vorgesehen. Bei einer solchen Konstruktion werden die Trokare 7 von dem Schiebeelement 11 sicher gehalten, auch wenn die Trokare 7 beispielsweise eine seitliche Ausnehmung von größer als 180° aufweisen und somit nicht ausreichend auf einer üblichen Trokarlanze gehalten werden können. Die Haltemittel 10 können insbesondere als unidirektionale Zähnchen ausgebildet sein.

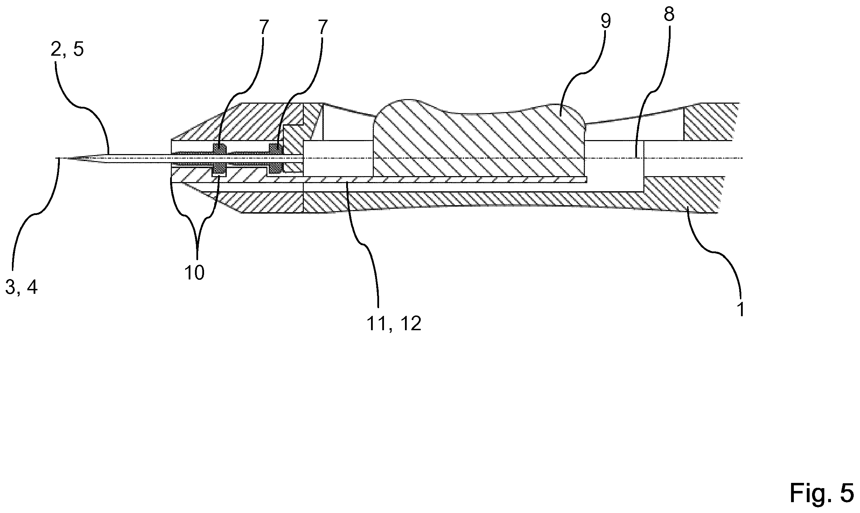

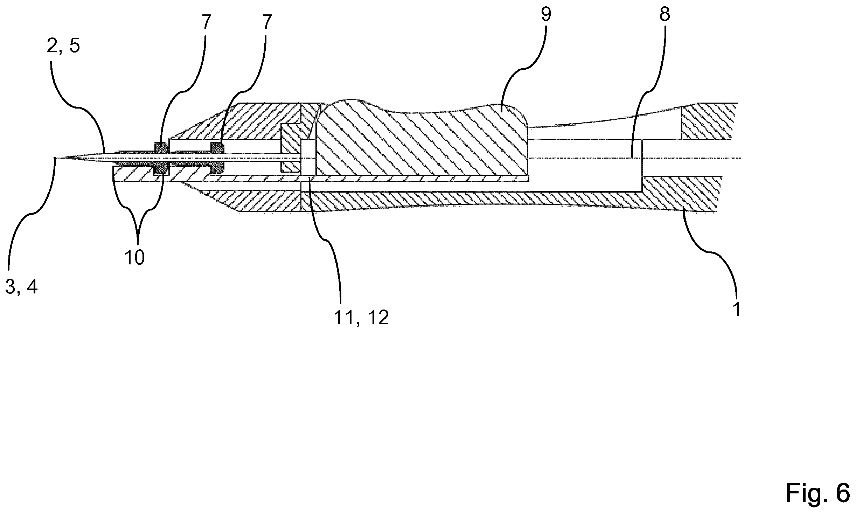

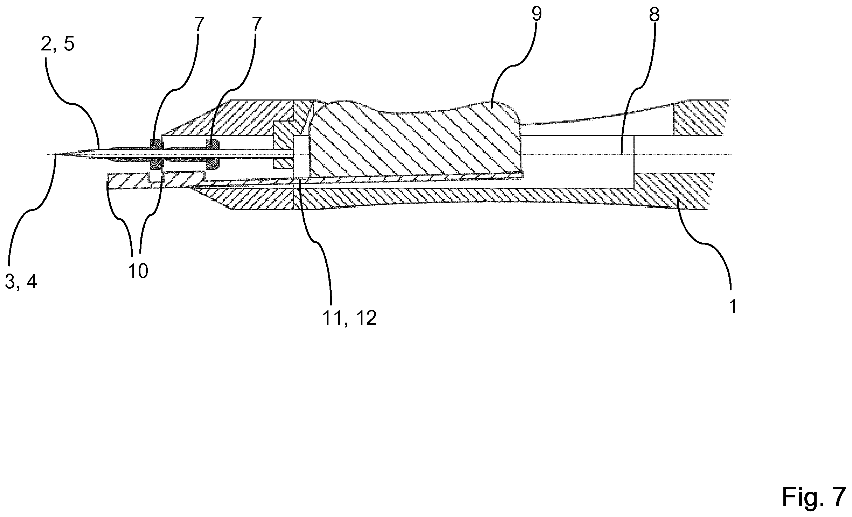

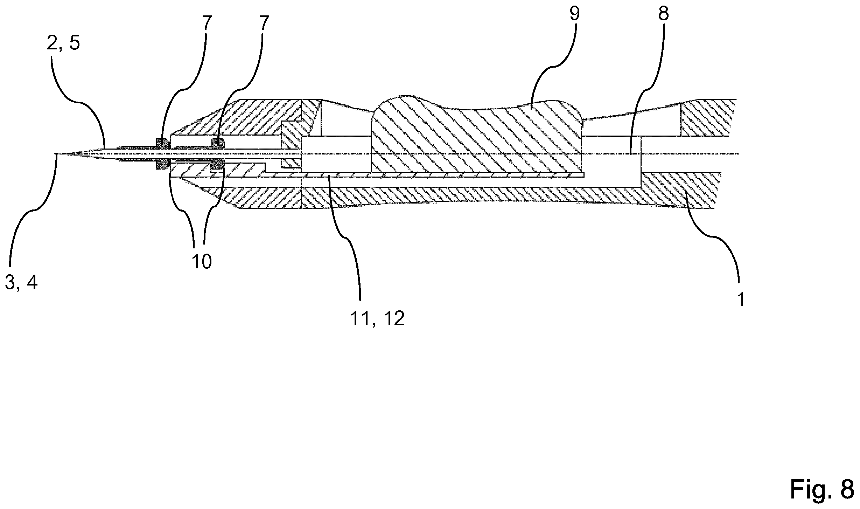

Mit der in Fig. 4 gezeigten Anordnung kann der Chirurg die erste Inzision mit der Spitze 4 an dem Auge anbringen und den vordersten Trokar 7 in diese Inzision einführen. Fig. 5 zeigt das Handgerät, nachdem der erste Trokar 7 in das Auge verbracht ist. Sodann kann der Chirurg die verbliebenen Trokare 7 mit dem Schiebeelement 11 in Richtung des distalen Endes 3 des Schafts 2 schieben (vgl. Fig. 6). In Fig. 7 ist deutlich zu erkennen, dass das Schiebeelement 11 zumindest geringfügig in Richtung von dem Schaft 2 wegbewegt werden kann, sodass das Schiebeelement 11 zurückgezogen und hinter den vorderen Trokar 7 verbringbar ist. Hierzu können die Haltemittel 10 als Zähnchen ausgebildet sein. Nachdem das Schiebeelement 11 zurückgezogen ist, befindet sich der vordere Trokar 7 in einer Position, in der eine weitere Inzision in dem Auge erzeugt werden kann und der Trokar 7 in das Auge eingebracht werden kann (vgl. Fig. 8). Der verbleibende Trokar 7 kann sodann über das Schiebeelement 11 nach vorne geschoben werden. Auf diese Weise ist es dem Chirurgen möglich, mehrere Trokare 7 nacheinander in das Auge einzubringen, ohne das Handgerät zwischenzeitlich ablegen zu müssen und/oder an dem Handgerät umgreifen zu müssen.

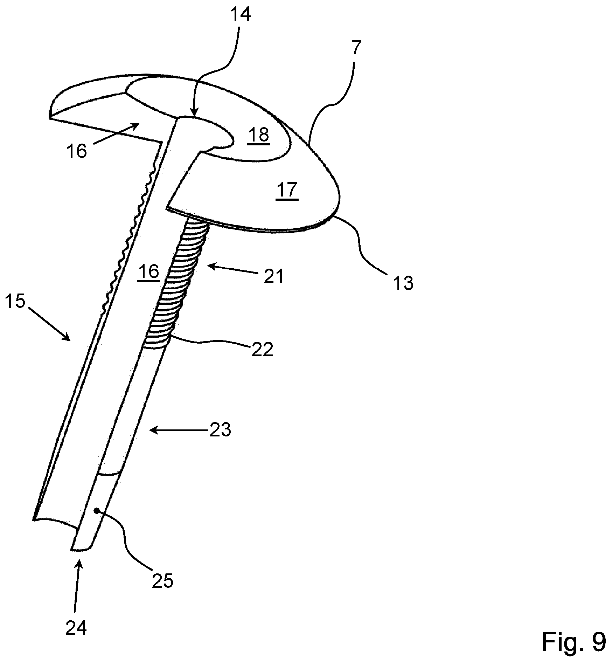

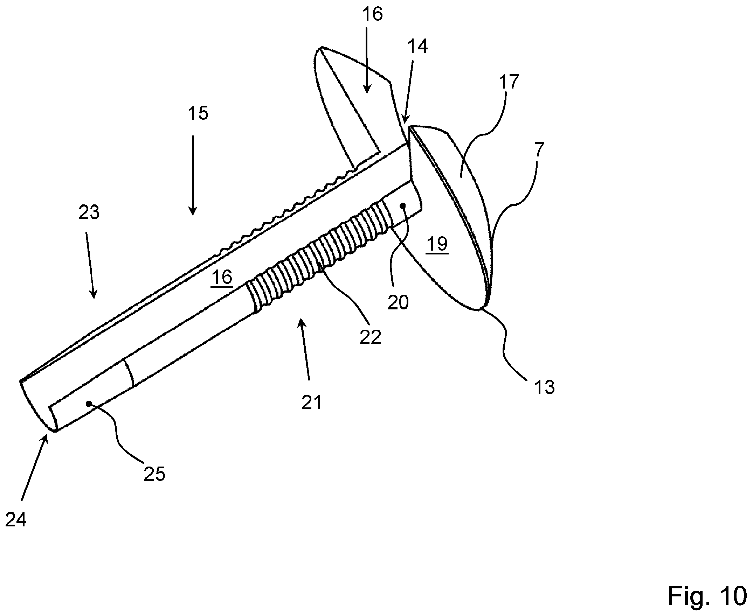

Die Fig. 9 und 10 zeigen ein Ausführungsbeispiel eines Trokars 7 zur Verwendung mit dem erfindungsgemäßen Handgerät. Der Trokar 7 umfasst einen Anschlusskörper 13, einen im Anschlusskörper 13 ausgebildeten Durchgang 14 und eine sich an den Durchgang 14 anschließende Sonde 15. Die Sonde 15 dient zum Einstecken in den menschlichen Körper bzw. in das Auge. Der Anschlusskörper 13 dient auf der der Sonde 15 zugewandten Seite zur Anlage außerhalb des Auges, nämlich zur Anlage an der Sklera. Auf der der Sonde 15 abgewandten Seite, d. h. anschlussseitig, dient der Anschlusskörper 13 zum Ankoppeln eines in der Fig. nicht gezeigten Schlauchs, zum Anstecken oder Einstecken eines Instruments, etc., wodurch eine Art Port gebildet ist.

Die Fig. 9 und 10 zeigen deutlich, dass der Anschlusskörper 13 äußerst flach ausgebildet ist, nämlich nur eine geringe Bauhöhe aufweist. Im Konkreten ist der Anschlusskörper 13 ähnlich einem runden Schraubenkopf gebildet, nämlich scheibenartig, tellerartig oder knopfartig. Anschlussseitig ist der Anschlusskörper 13 abgerundet und anlageseitig flach ausgebildet, wobei der Anschlusskörper 13 anlageseitig auch gewölbt oder konkav ausgebildet sein kann, nämlich zur Begünstigung der Anlage an der Oberfläche des Auges.

Der Trokar ist sowohl im Anschlusskörper 13 als auch im Durchgang 14 und in der Sonde 15 mit einer Ausnehmung 16 ausgestattet, die im Anschlusskörper 13 im Sinne eines Teilkreissegments ausgeführt ist. Dadurch ist es möglich, dass bereits beim Einstecken des Trokars in das Augengewebe dieses die Ausnehmung 16 ausfüllt und eine Abdichtung aus Gewebe bildet.

Der Anschlusskörper 13 hat auf seiner der Sonde 15 abgewandten Oberseite eine konvex gewölbte Grundform 17, in der eine konkave Wölbung 18 im Sinne einer Einführhilfe ausgebildet ist. Der gesamte Anschlusskörper 13 ist durch die Ausnehmung 16 unterbrochen, nicht nur die gewölbte Grundform 17, sondern auch die konkave Wölbung 18.

Auf der Unterseite, nämlich auf der Seite der Sonde 15, hat der Anschlusskörper 13 eine flache bzw. ebene Fläche 19, die zur besseren Anpassung an die Oberfläche des Auges konkav ausgeführt sein kann.

Der Anschlusskörper 13 mündet über den Durchgang 14 in die Sonde 15, durch die hindurch ein Instrument, beispielsweise ein Lichtleiter, in den Körper einführbar ist.

Die Sonde 15 schließt sich an die Unterseite des Anschlusskörpers 13 an, bei dem in den Figuren gezeigten Ausführungsbeispiel mit einem kurzen ersten Bereich 20, der an der Oberfläche glatt ausgeführt ist. Durch diese Maßnahme soll das Gewebe im eingesteckten Zustand des Trokars geschont werden.

An den ersten glatten Bereich 20 schließt sich ein zweiter Bereich 21 an, der mit Drehriefen 22 oder mit einem Außengewinde versehen ist. Durch diese Maßnahme soll ein unbeabsichtigtes Herausgleiten des Trokars aus dem Augengewebe vermieden werden.

An den zweiten Bereich 21 schließt sich ein dritter Bereich 23 mit abermals glatter Oberfläche an, der sich zum distalen Ende 24 hin verjüngt. Dieser verjüngte Bereich 25 ist dadurch geschaffen, dass die Wandung zum distalen Ende 24 hin in der Dicke abnimmt, nämlich von außen nach innen gerichtet, so dass das distale Ende 24 der Sonde 15 das Einführen in die Inzision begünstigt. Das distale Ende 24 kann auch abgeschrägt ausgebildet sein, so dass die von der Ausnehmung 16 abgewandte Wandung der Sonde 15 ein mehr oder weniger spitz zulaufendes distales Ende bildet. Dieses Ende kann auch einen Radius mit einer Abrundung aufweisen. Dieser Bereich hat eine glatte Oberfläche, mit möglichst sehr guter Gleiteigenschaft gegenüber dem Augengewebe.

Es sei angemerkt, dass der gesamte Trokar 7, umfassend den Anschlusskörper 13 und die Sonde 15, aus Kunststoff oder Metall gefertigt ist. Bei einer Ausführung aus Metall sind biokompatible Werkstoffe von Vorteil, beispielsweise Titan oder Titanlegierungen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel der erfindungsgemäßen Vorrichtung lediglich zur Erörterung der beanspruchten Lehre dient, diese jedoch nicht auf das Ausführungsbeispiel einschränkt.

Die Erfindung wird im beigefügten Anspruchssatz definiert.

### Bezugszeichenliste

- 1: Griffstück
- 2: Schaft
- 3: distales Ende (Schaft)
- 4: Spitze
- 5: Magazineinrichtung
- 6: Aufnahme
- 7: Trokar
- 8: Mittelachse
- 9: Betätigungselement
- 10: Haltemittel
- 11: Verschiebeelement
- 12: Verbringeinrichtung
- 13: Anschlusskörper
- 14: Durchgang
- 15: Sonde
- 16: Ausnehmung
- 17: Grundform
- 18: Wölbung
- 19: Fläche
- 20: erster Bereich
- 21: zweiter Bereich
- 22: Drehriefen
- 23: dritter Bereich
- 24: distales Ende (Sonde)
- 25: verjüngter Bereich

## Patentansprüche

1. Ophthalmologisches Handgerät zum Einbringen einer Inzision in das menschliche oder tierische Auge mit einem Griffstück (1) und einem Schaft (2), wobei an einem distalen Ende (3) des Schafts (2) eine Spitze (4) ausgebildet ist, wobei eine Magazineinrichtung (5) zum Vorhalten von mindestens zwei Trokaren (7) angeordnet ist, wobei die Trokare (7) hintereinander auf dem Schaft (2) aufgesteckt sind, so dass der Schaft (2) die Magazineinrichtung (5) bildet, wobei die Trokare (7) über eine Verbringeinrichtung (12) in Richtung des dem Auge zugewandten Endes des Griffstücks (1) verbringbar sind, und wobei die Verbringeinrichtung (12) ein Schiebeelement (11) mit einem Haltemittel (10) für einen Trokar (7) aufweist,
**dadurch gekennzeichnet, dass** sich das Schiebeelement (11) seitlich versetzt von der Mittelachse (8) des Schafts (2) erstreckt.

2. Ophthalmologische Handgerät nach Anspruch 1, **dadurch gekennzeichnet, dass** von der Magazineinrichtung (5) mindestens drei Trokare (7) aufnehmbar sind.

3. Ophthalmologisches Handgerät nach einem der Ansprüche 1 bis 2, **dadurch gekennzeichnet, dass** das Schiebeelement (11) in Richtung der Mittelachse (8) des Schafts (2) vorgespannt ist.

4. Ophthalmologisches Handgerät nach einem der Ansprüche 1 bis 3, **dadurch gekennzeichnet, dass** das Schiebeelement (11) im Wesentlichen in Radialrichtung von dem Schaft (2) weg bewegbar ist.

5. Ophthalmologische Handgerät nach einem der Ansprüche 1 bis 4, **dadurch gekennzeichnet, dass** die Spitze (4) des Schafts (2) eine seitliche Ausnehmung aufweist, so dass die Spitze (4) einen von einem runden Querschnitt abweichenden Querschnitt aufweist.

6. Ophthalmologische Handgerät nach einem der Ansprüche 1 bis 5, **dadurch gekennzeichnet, dass** die Spitze (4) des Schafts (2) einen MVR-Schliff aufweist.

7. Set bestehend aus einem ophthalmologischen Handgerät nach einem der Ansprüche 1 bis 6 und mindestens zwei Trokaren (7), wobei die Trokare (7) in und/oder an der Magazineinrichtung (5) anordenbar sind.

## Claims

1. Ophthalmological hand-held device for introducing an incision into a human or animal eye having a handle piece (1) and a shaft (2), wherein at a distal end (3) of the shaft (2) a tip (4) is formed, wherein a storage device (5) for storing at least two trocars (7) is arranged, wherein the trocars (7) are fitted one behind the other on the shaft (2) so that the shaft (2) forms the storage device (5), wherein the trocars (7) can be moved by means of a transfer device (12) in the direction of the end of the handle piece (1) facing the eye, and wherein the transfer device (12) has a sliding element (11) having a retention means (10) for a trocar (7), **characterised in that** the sliding element (11) extends in a laterally offset state from the centre axis (8) of the shaft (2).

2. Ophthalmological hand-held device according to claim 1, **characterised in that** at least three trocars (7) can be received by the storage device (5).

3. Ophthalmological hand-held device according to either claim 1 or 2, **characterised in that** the sliding element (11) is pretensioned in the direction of the centre axis (8) of the shaft (2).

4. Ophthalmological hand-held device according to any one of claims 1 to 3, **characterised in that** the sliding element (11) can be moved substantially in the radial direction away from the shaft (2).

5. Ophthalmological hand-held device according to any one of claims 1 to 4, **characterised in that** the tip (4) of the shaft (2) has a lateral recess so that the tip (4) has a cross-section which differs from a round cross-section.

6. Ophthalmological hand-held device according to any one of claims 1 to 5, **characterised in that** the tip (4) of the shaft (2) has an MVR blade.

7. Set comprising an ophthalmological hand-held device according to any one of claims 1 to 6, and at least two trocars (7), wherein the trocars (7) can be arranged in and/or on the storage device (5).

## Revendications

1. Instrument ophtalmologique portatif destiné à pratiquer une incision dans l'oeil humain ou animal, comprenant une poignée (1) et une tige (2), une pointe (4) étant formée à une extrémité distale (3) de la tige (2), un dispositif de magasin (5) étant agencé pour contenir au moins deux trocarts (7), les trocarts (7) étant enfichés l'un derrière l'autre sur la tige (2) de telle sorte que la tige (2) forme le dispositif de magasin (5), les trocarts (7) pouvant être déplacés vers l'extrémité de la poignée (1) tournée vers l'oeil par l'intermédiaire d'un dispositif de déplacement (12), et le dispositif de déplacement (12) comportant un élément coulissant (11) avec un moyen de retenue (10) pour un trocart (7), **caractérisé en ce que** l'élément coulissant (11) s'étend de manière décalée latéralement par rapport à l'axe central (8) de la tige (2).

2. Instrument ophtalmologique portatif selon la revendication 1, **caractérisé en ce que** le dispositif de magasin (5) peut recevoir au moins trois trocarts (7).

3. Instrument ophtalmologique portatif selon l'une des revendications 1 à 2, **caractérisé en ce que** l'élément coulissant (11) est précontraint dans la direction de l'axe central (8) de la tige (2).

4. Instrument ophtalmologique portatif selon l'une des revendications 1 à 3, **caractérisé en ce que** l'élément coulissant (11) est déplaçable essentiellement dans la direction radiale à partir de la tige (2).

5. Instrument ophtalmologique portatif selon l'une des revendications 1 à 4, **caractérisé en ce que** la pointe (4) de la tige (2) présente un évidement latéral, de sorte que la pointe (4) présente une section transversale qui s'écarte d'une section transversale ronde.

6. Instrument ophtalmologique portatif selon l'une des revendications 1 à 5, **caractérisé en ce que** la pointe (4) de la tige (2) présente une coupe MVR.

7. Ensemble composé d'un instrument ophtalmologique portatif selon l'une des revendications 1 à 6 et d'au moins deux trocarts (7), les trocarts (7) pouvant être disposés dans et/ou sur le dispositif de magasin (5).
